# EUROPEAN PATENT APPLICATION

(11) **EP 1 887 487 A2**
(43) Date of publication of application: **13.02.2008**
(21) Application number: 07252573.6
(22) Date of filing: 25.06.2007
(51) Int. Cl.: G06F 19/00

(54) **Use of archived data in interpretation of medical images**

(30) Priority: 07.08.2006 US 500027
(71) Applicant: iCad, Inc., Beavercreek, Ohio 45431 (US)
(72) Inventor: Worrell, Steve, Beavercreek, Ohio 45440 (US)
(74) Representative: Cummings, Sean Patrick

(57) **Abstract**

Retrieval of archival data from an application such as, for example, a CAD system, to aid in the interpretation of current data using the same application is disclosed. The data may comprise of medical imagery. The archival data may be used to transform a current set of images to be more consistent with a reference set of images to facilitate comparison between the sets of images. The archival data may also be used to influence markings and assessment of the current set of images. Additionally, the archival data may be used in conjugation with prior computer aided-detection markings or user identified regions to aid in the assessment of regions of interest identified in the current examination.

## Description

### BACKGROUND OF THE INVENTION

The present invention is generally related to the interaction of an application with an archival system and, in particular, relates to the interaction of an application with an archival system to enhance the user display, computer aided-detected (CAD) markings, or provide exemplars to the user consistent with a region identified by an application or user.

CAD systems have become widely available to assist readers of medical imagery, such as radiologists. These CAD systems are commercially available to assist radiologists in the detection of signs of cancer in mammographic and thoracic imagery by highlighting regions of possible malignancies. In other words, the CAD systems are, in essence, a second reader of the medical imagery for the radiologist.

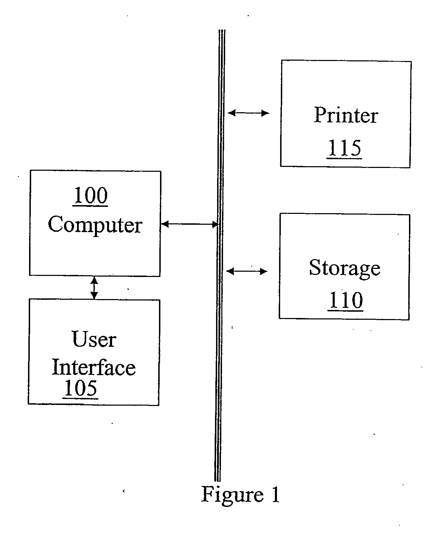

Figure 1 illustrates a high level block diagram of a typical CAD system. Digital medical images are produced by methods well known in the art and feed into the CAD system. A radiology technologist uses interface devices 105 such as a keyboard, mouse, touch screen, bar code reader or speech recognition application to control the CAD system operation and input patient identification information into an electronic system 100, such as a computer. The medical images are analyzed for signs of cancer by algorithms in the CAD system.

The CAD system produces a visual or textual indication of the location and type of cancer indicator suspected. The output is typically either a printed page from a printer 115 or electronic file stored in storage 110 consisting of the digital medical images with suspicious regions highlighted by markers; different marker styles are used to denote different indicators of cancer. When the CAD output is a printed page, it may be stored with a hardcopy of the medical images. When the CAD output is an electronic file, it is stored, or archived, and recorded such that it may be recalled using patient identification information and subsequently printed or displayed on a monitor, 120. A radiologist then uses the CAD output during an interpretation phase.

It is common for radiologists to use the stored prior films for comparison to the current examination films to aid in identifying any change between examinations. Historically, image processing, displays, and CAD systems have operated on a single image and/or patient current examinations that have not been in any way adaptive based on the existing archived patient data. However, to aid in identifying change between examinations, it is desirable to have images as consistent as possible with respect to scale and intensity distribution. Additionally, the CAD markings on the current imagery can be influenced by the archived data stored in the form of patient records (e.g., prior examinations, patient history) as well as medical images from the same type of application or from other modalities such as, for example, X-ray images and computed tomography (CT) images.

Therefore, there is a need for the development of techniques that leverage the increasing volume of data being stored in archival systems to aid in improving image quality, image consistency between different acquisition devices used on the same patient, CAD markings and/or user assessment (or understanding) of the relevance of a subsequent identified region in later medical imagery.

### BRIEF SUMMARY OF THE INVENTION

According to the present invention, archival data from an application such as, for example, a CAD system, may be retrieved to aid in the interpretation of current data from the same application. The data may comprise of medical imagery.

In accordance with one embodiment of the present invention, archival data may be used to transform a current set of images to be more consistent with a reference set of images in order to facilitate comparisons between the two sets of images.

In accordance with another embodiment of the present invention, archival data may be used to influence detection markings and assessment of a current set of images.

In accordance with yet another embodiment of the present invention, archival data may be used in conjugation with prior computer aided-detection markings or user identified regions to aid in the assessment of region of interest identified in a current examination.

Accordingly, it is a feature of the embodiments of the present invention to optimize medical images based on a reference set of images to facilitate comparison between the current set of images and the reference set of images.

It is another feature of the embodiments of the present invention to use archived data, imagery, and patient reports to influence the CAD markings of the current imagery of that patient.

Still another feature of the embodiments of the present invention is to use archived data to identify exemplars similar to a region of interest currently marked by the CAD system or identified by the user as a region of interest.

Other features of the embodiments of the present invention will be apparent in light of the description of the invention embodied herein.

### BRIEF DESCRIPTION OF THE SEVERAL VIEWS OF THE DRAWINGS

The following detailed description of specific embodiments of the present invention can be best understood when read in conjunction with the following drawings, where like structure is indicated with like reference numerals and in which:
Fig. 1 is a high level block diagram of a typical CAD system;
Fig. 2 is a block diagram illustrating general overview according to an embodiment of the present invention.

### DETAILED DESCRIPTION

In the following detailed description of the embodiments, reference is made to the accompanying drawings that form a part hereof, and in which are shown by way of illustration, and not by way of limitation, specific embodiments in which the invention may be practiced. It is to be understood that other embodiments may be utilized and that logical, mechanical and electrical changes may be made without departing from the spirit and scope of the present invention.

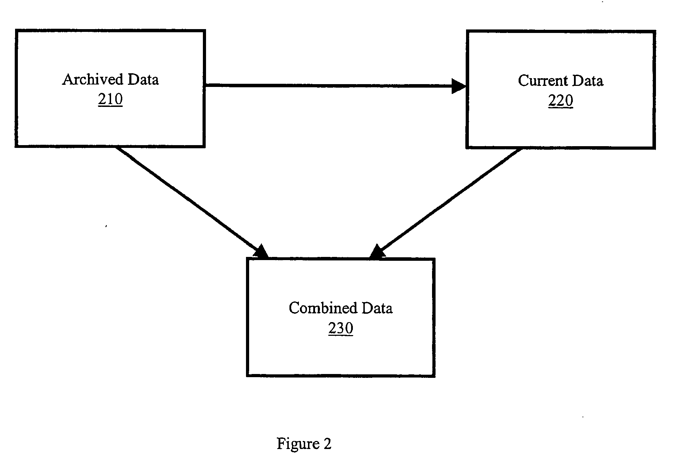

In one embodiment, radiologists can use archived data from prior films for comparison to current examination films to aid in identifying changes between examinations. In order to more easily identify any changes between examinations, it is desirable to have the images be as consistent as possible with respect to scale and intensity distribution. To achieve this objective and referring to Fig. 2, archived data 210 can be "fetched," or retrieved from storage 110 and used as the basis for establishing transformations to make images 230 from different acquisition devices and/or times consistent with a set of reference images 220. For example, prior examination images are retrieved from an archive and the intensity distribution and scale of the current examination are then matched to archived data using image processing techniques such as histogram matching and re-sampling.

The data 210 is retrieved from an archive, based on a unique patient identifier that is stored with the data, and used to aid in transforming a set of images from the current examination 220 to be more consistent with a reference set of images. The transformed data 230 then can be displayed on a monitor or printed for the user to review. The archived data 210 may be in the form of images, signals and textual records. Using the archived data 210 helps optimize comparisons between the set of current images and the reference set of images.

In another embodiment, the CAD markings of the current examination can be influenced by archived data. The archived data can be in the form of patient records such as, for example, prior examinations and patient histories as well as images from the same application or other modalities, such as, for example, X-ray images or CT scans. The archived data may be used to adapt thresholds, influence a-priori probabilities of disease, or identify regions of interest in the imagery of the current examination that have been identified by an expert or a CAD system as being suspicious or that have been identified through image coordinates. Indications of suspicious could include areas identified as biopsied, requiring additional views and identified for surgical procedures or marked by the CAD system in previous examinations.

The data is retrieved from an archive, based on a unique patient identifier that is stored with the data, and is used to influence markings and assessment of the current examination. The influenced data can then be displayed on a monitor, or printed, for the user to review. The archived data may be in the form of images, signals and textual records. Using the archived data, imagery and reports currently existing for a patient can help influence and strength the markings of the CAD system for the current examination of the patient.

In yet another embodiment, images from the archive can be identified that have regions of interest that are similar to a region identified by the CAD system to be of interest in order to aid the user in understanding the basis of a current CAD marking. Alternatively, the regions may be identified by the user as being of interest. Regions of interest may be identified using a suitable similarity metric. Given a region of interest identified as "similar", users may be presented with those regions of interest associated with those regions of interest with markers indicated the specific regions of interest are "similar." Alternatively, the users may be presented with the entire image with associated regions of interest marked. These similar regions of interest may form the basis for user to easily dismiss the current mark due to a disparity between the extracted regions of interest from the archival data or may increase the level of suspicion for the user due to the knowledge that the regions of interest extracted from an archive were found to be disease and are similar visually to the marked region of interest of the current examination.

The data is retrieved from an archive based on a similarity metric that is deemed to be consistent with a region of interest identified by the CAD system or a user and is subsequently displayed to the user to aid in the assessment of a region of interest identified in the current examination. Alternatively, the archived data is retrieved based on unique patient identifier that is stored with the data in the archive. In another embodiment, a similarity metric and a unique patient identifier are both used to retrieve the data from the archive. The similar archived marks can then be displayed with the currently identified regions of interest on a monitor for the user to review. Alternatively, the similar archived marks and the currently identified regions of interest can both be printed as output for the current examination. Using the archived data facilitates the identification of exemplars similar to a region of interest marked by a CAD system or identified by the user as a region of interest.

It is noted that terms like "preferably," "commonly," and "typically" are not utilized herein to limit the scope of the claimed invention or to imply that certain features are critical, essential, or even important to the structure or function of the claimed invention. Rather, these terms are merely intended to highlight alternative or additional features that may or may not be utilized in a particular embodiment of the present invention.

Having described the invention in detail and by reference to specific embodiments thereof, it will be apparent that modifications and variations are possible without departing from the scope of the invention defined in the appended claims. More specifically, although some aspects of the present invention are identified herein as preferred or particularly advantageous, it is contemplated that the present invention is not necessarily limited to these preferred aspects of the invention.

## Claims

1. A method of using archival data in interpretation of current data from medical applications, the method comprising:
acquiring images from a medical application for a patient;
retrieving archival data for the same patient; and
using archived data to transform acquired images so that the acquired images are consistent with previous images from the same patient.

2. The method of claim 1, wherein the acquired images are matched to the previous images through image processing techniques using the archived data.

3. The method of claim 2, wherein the image processing techniques include histogram matching, re-sampling, or combinations thereof.

4. The method of any preceding claim, wherein intensity distribution and scale of the acquired images are matched to the intensity distribution and scale of the previous images through image processing techniques using the archived data.

5. The method of any preceding claim, wherein the archived data comprises images, signals, textual records and combinations thereof.

6. The method of any preceding claim, wherein the archival data is retrieved based on a unique identifier for the patient stored with the archived data.

7. The method of any preceding claim, further comprising:
displaying the transformed acquired images for review by a user.

8. A method of using archival data in interpretation of current data from medical applications, the method comprising:
acquiring images from a medical application for a patient;
processing the acquired images with a computer-aided detection system to detect regions of interest;
retrieving archival data for the same patient; and
using archived data to influence detection markings and assessment on the acquired images.

9. The method of claim 8, further comprising:
using the archived data to adapt thresholds in the acquired images.

10. The method of claim 8 or claim 9, further comprising:
using the archived data to influence a-priori probabilities of disease in the acquired images.

11. The method of any of claims 8 to 10, further comprising:
using the archived data to identify regions of interest in the acquired images that have been identified as being suspicious in the archived data.

12. The method of claim 11, wherein suspicious areas in the archived data comprises biopsied areas, areas requiring additional views, areas identified as needing surgical procedures, computer-aided detected marks or combinations thereof.

13. The method of claim 11 or claim 12, wherein suspicious areas in the archived data are identified by an expert reader.

14. The method of any of claims 11 to 13, wherein suspicious areas in the archived data are identified by a computer-aided detection system.

15. The method of any of claims 8 to 14, further comprising:
using the archived data to identify regions of interest in the acquired images that have been identified through image coordinates in the archived data.

16. The method of any of claims 8 to 15, wherein the archived data comprises images, signals, textual records and combinations thereof.

17. The method of any of claims 8 to 16, wherein the archived data comprises patient records.

18. The method of claim 17, wherein the patient records comprise prior examinations, patient histories or combinations thereof.

19. The method of any of claims 8 to 18, wherein the archived data comprises images produced from the same application.

20. The method of any of claims 8 to 19, wherein the archived data comprises images produced from the other modalities.

21. The method of claim 20, wherein the other modalities include X-ray machines and computed tomography.

22. The method of any of claims 8 to 21, wherein the archival data is retrieved based on a unique identifier for the patient stored with the archived data.

23. The method of any of claims 8 to 22, further comprising:
displaying the influenced acquired images for review by a user.

24. A method of using archival data in interpretation of current data from medical applications, the method comprising:
acquiring images from a medical application for a patient;
processing the acquired images with a computer-aided detection system to detect regions of interest;
retrieving prior identified regions of interest from archival data for the same patient; and
using the prior identified regions of interest from archived data to aid in assessing the regions of interest in the acquired images that are similar to the prior identified regions of interest from archived data.

25. The method of claim 24, further comprising:
marking the prior identified regions of interest that are deemed similar to the regions of interest in the acquired images; and
displaying the marked prior identified regions of interest on the acquired images.

26. The method of claim 24 or claim 25, further comprising:
marking the prior identified regions of interest that are deemed similar to the regions of interest in the acquired images; and
displaying the entire archived image with the marked prior identified regions of interest.

27. The method of any of claims 24 to 26, further comprising:
dismissing the regions of interest in the acquired images due to the lack of similarity with the prior identified regions of interest.

28. The method of any of claims 24 to 27, further comprising:
increasing the level of suspicion regarding the regions of interest in the acquired images due to the level of similarity with the prior identified regions of interest.

29. The method of any of claims 24 to 28, further comprising:
increasing the level of suspicion regarding the regions of interest in the acquired images due to the level of similarity with the prior identified regions of interest and the knowledge that the prior identified regions of interest were found to be disease.

30. The method of any of claims 24 to 29, wherein the prior identified regions of interest from archived data are identified by a computer-aided detection system.

31. The method of any of claims 24 to 30, wherein the prior identified regions of interest from archived data are identified by a user

32. The method of any of claims 24 to 31, wherein the archival data is retrieved based on a similarity metric.

33. The method of any of claims 24 to 32, wherein the archival data is retrieved based on a unique identifier for the patient stored with the archived data.

34. The method of any of claims 24 to 33, wherein the archival data is retrieved based on a similarity metric and a unique identifier for the patient stored with the archived data.
